# FASCICULE DE BREVET EUROPEEN

(11) **EP 3 092 218 B1**
(45) Date de publication et mention de la délivrance du brevet: **09.03.2022**
(21) Numéro de dépôt: 14701813.9
(22) Date de dépôt: 07.01.2014
(51) Int. Cl.: C07D 233/64, C07C 227/40, C07D 209/20, B01J 39/05, B01J 39/20

(54) **PROCÉDÉ DE PURIFICATION D'ACIDES AMINÉS AROMATIQUES**
VERFAHREN ZUR REINIGUNG VON AMINOSÄUREN
PROCESS FOR THE PURIFICATION OF AROMATIC AMINOACIDS

(43) Date de publication de la demande: 16.11.2016
(73) Titulaire: Novasep Process Solutions, 01700 Saint-Maurice-de-Beynost (FR)
(72) Inventeur: BRICHANT, Damien, 56370 Sarzeau (FR); SCHAB, Frédéric, 69007 Lyon (FR); BAUDOUIN, Stanislas, 17180 Périgny (FR); ORIEZ, Vincent, 78600 Maisons-Laffitte (FR)
(74) Mandataire: Bandpay & Greuter
(86) Numéro de dépôt international: PCT/FR2014/050015
(87) Numéro de publication internationale: WO 2015/104464

(56) Documents cités:
- EP-A1- 0 027 874
- EP-A1- 1 106 602
- EP-A2- 0 200 944
- FR-A1- 2 557 872
- US-A- 5 300 653
- US-A1- 2009 130 723

## Description

### DOMAINE DE L'INVENTION

La présente invention concerne un procédé de purification d'acides aminés aromatiques, c'est-à-dire d'acides aminés comportant un cycle aromatique, et en premier lieu le tryptophane, la phénylalanine, la tyrosine, et l'histidine.

### ARRIERE-PLAN TECHNIQUE

Les acides aminés aromatiques peuvent être produits par fermentation, par enzymation ou par voie synthétique.

Le tryptophane, la phénylalanine et l'histidine (pour les nourrissons) font partie de la famille des acides aminés essentiels : à ce titre, ils sont utilisés en tant que compléments nutritifs.

Le moût d'acide aminé produit par voie fermentaire, synthétique ou enzymatique contient des impuretés parmi lesquelles des sucres et substrats résiduels, des minéraux, des pigments. Ces impuretés doivent être éliminées pour atteindre le degré de pureté requis pour son utilisation commerciale.

Diverses méthodes de purification ont été développées pour purifier les acides aminés aromatiques. Elles sont basées sur les technologies dominantes suivantes : cristallisation, extraction liquide / liquide et fixation sur résine.

La purification par cristallisation est mise en œuvre en voie aqueuse ou solvant. Des étapes de de prétraitement avant cristallisation sont parfois prévues, de manière à augmenter la pureté des cristaux. Il peut s'agir d'une étape de purification sur charbon actif (documents CN 101245047 et CN 102146052) ; d'une étape de filtration membranaire sur filtre inox (document CN 101376646) ; d'une étape d'ultrafiltration (document CN 101691349) ; ou d'une combinaison de plusieurs filtrations successives pour éliminer plusieurs familles d'impuretés, par exemple un enchaînement microfiltration / ultrafiltration / nanofiltration (document CN 101812009).

Des solvants sont parfois ajoutés lors de la cristallisation pour atteindre de meilleurs degrés de pureté, tels que de l'acide acétique (documents JP 2097801 et EP 1756055), ou un alcool aliphatique (document EP 0274728). L'utilisation de ces solvants entraîne une augmentation des coûts de production, et des coûts de traitement ou de recyclage des liqueurs mères.

Une deuxième voie de purification consiste à procéder à une extraction liquide / liquide de l'acide aminé. Ainsi, il a été proposé une purification du tryptophane utilisant des tensioactifs pour former une phase micellaire inverse (document CN 102382030), ou encore du toluène (document EP 0299715). Ces techniques ne sont jamais suffisantes en tant que telles pour atteindre de hauts degrés de pureté. Des étapes de pré- et de post-traitement sont nécessaires. De plus, la mise en œuvre de volumes importants de solvants est économiquement peu intéressante pour traiter des volumes industriels d'acides aminés à l'échelle de plusieurs kilotonnes / an / unité.

Enfin, une troisième voie propose un traitement sur résine polymérique.

Dans les documents CN 101812488, CN 101948420 et US 2013/0079527, la purification repose sur une capture et fixation de l'acide aminé sur une résine adsorbante (donc neutre), puis une élution à l'éthanol (dans les deux premiers documents) ou avec un sel (dans le troisième document).

Le document FR 2581654 décrit l'adsorption du tryptophane sur une résine cationique forte polystyrénique faiblement réticulée. Le composé cible est précipité, puis dissout dans une solution acide, notamment une solution d'acide sulfurique ou chlorhydrique ayant un pH de 1 à 2. La solution acide est ensuite mise en contact avec une résine échangeuse de cations, qui est rincée à l'eau puis s'ensuit une élution par une base. Ce procédé de type « batch » est la voie couramment en usage aujourd'hui pour une mise en œ uvre industrielle.

Le document FR 1502814 décrit l'adsorption du tryptophane sur une résine cationique forte ou sur une résine anionique forte. Dans ces deux cas, la résine sélectionnée présente un faible taux de réticulation. Dans le cas d'une résine cationique forte, le pH du mélange contenant le tryptophane est ajusté à 2 avant la mise en contact avec la résine. L'élution est effectuée en deux temps: d'abord une élution par une solution saline neutre pour éliminer des acides aminés polluants, puis une élution par une solution basique pour désorber le tryptophane. Il s'agit d'un procédé nécessitant deux éluants distincts et des quantités importantes de régénérants.

L'inconvénient des traitements sur résines décrits ci-dessus est de nécessiter une consommation importante de régénérant chimique concentré (usuellement 2 à 10 % en masse) pour éluer l'acide aminé après fixation. Une quantité d'effluents équivalente à celle de régénérant est également engendrée.

Le document US 5300653 décrit la séparation sur colonne unique en batch d'au moins un acide aminé aromatique d'autres acides aminés non aromatiques sur une résine cationique forte de type gel initialement convertie sous forme saline avec un métal alcalin. La solution contenant l'acide aminé présente de préférence un pH de 2 à 12. L'acide aminé est adsorbé lors du passage de la solution sur la résine, puis il est désorbé avec de l'eau dépourvue d'acide ou de base ou un solvant organique.

Un inconvénient de cette méthode est que le mode d'élution ne permet pas de maîtriser la forme ionique de la résine. En effet, les espèces cationiques présentes dans la solution à traiter sont susceptibles de s'échanger avec le métal alcalin, sans qu'aucun éluant ne permette de maintenir la forme ionique initiale. Les performances de séparation entre les acides aminés peuvent être affectées par ces mécanismes d'échange d'ions intempestifs : par conséquent, les rendements et puretés en acides aminés produits risquent de diminuer ou de varier de manière incontrôlée dans le temps.

Le document EP 0200944 décrit un procédé de purification du tryptophane vis à vis de rindole. Le pH du fluide à purifier est ajusté à 4, puis après des prétraitements, il est passé sur une résine cationique. Après un rinçage à l'eau, le tryptophane est élué avec une solution d'ammoniaque à 10 %.

Le document FR 2557872 décrit un procédé d'isolation du tryptophane d'un mélange réactionnel comprenant un microorganisme, dans lequel, notamment, une solution de tryptophane est passée sur une résine échangeuse de cations fortement acide sans ajustement préalable de son pH, un lavage à l'eau est réalisé et le tryptophane est ensuite élué avec une solution aqueuse d'ammoniac.

Le document EP 0027874 concerne un procédé de purification d'un acide aromatique par adsorption sur une résine échangeuse d'ions fortement acide et élution à l'aide d'une solution aqueuse d'ammoniac. Aucun n'aiustement du pH de la solution à passer sur la résine n'est indiqué.

Le document US 2009/0130723 concerne un procédé de purification de l'histidine d'une culture microbienne dans lequel la culture est chargée sur une colonne remplie de particules de 350 um ou plus d'un support qui est de préférence une résine échangeuse de cations acide forte. L'histidine est ensuite éluée avec un éluant, qui peut être une solution aqueuse d'ammoniac.

La majorité de ces traitements sur résine repose sur une mise en œuvre de type « *batch* », c'est-à-dire discontinue. Les résines sont installées en colonne, et les éluants successifs (moût contenant l'acide aminé ; eaux de rinçage ; solutions régénérantes - salines, basiques, acides ou alcooliques) sont percolés successivement à travers la colonne.

Toutefois le document US 2013/0079527 susmentionné décrit une séparation semi-continue sur résine adsorbante de type XAD7, dans un système de chromatographie à lit mobile simulé (SMB, pour Simulated Moving Bed) comportant trois zones de séparation. Le système est élué à l'eau ou au sel. Cette méthode présente l'inconvénient d'opérer sur une résine adsorbante, donc une résine plus coûteuse que des résines échangeuses d'ions. Les résines adsorbantes ont également tendance à s'encrasser rapidement. Leur utilisation nécessite un protocole de nettoyage à l'alcool, à la base et à l'acide pour éliminer les impuretés responsables de l'encrassement, ce qui accroît les contraintes opératoires en termes de coûts et d'équipements installés.

Le document EP 1106602 décrit une méthode de purification d'un acide aminé basique, en particulier la lysine, à l'aide d'un matériau chromatographique échangeur de cations, l'éluant étant un éluant aqueux basique, de préférence une solution aqueuse d'ammoniac. L'histidine est mentionnée en tant qu'acide aminé basique.

Ainsi, il existe un besoin de mettre au point un procédé de purification d'acide aminé aromatique amélioré, permettant notamment une séparation entre l'acide aminé cible et des impuretés telles que les sels, les sucres, les couleurs, avec une consommation minime de produits chimiques.

### RESUME DE L'INVENTION

L'invention concerne en premier lieu un procédé de purification d'un acide aminé à partir d'un mélange de départ, dans lequel l'acide aminé comporte un cycle aromatique et présente une constante d'acidité Ka, le procédé comprenant :
- une première étape de mise en contact du mélange de départ avec une résine cationique forte, à un pH supérieur ou égal à pKa ;
- une deuxième étape d'élution avec une solution aqueuse d'éluant présentant un pH supérieur au pH de la première étape, permettant de collecter un flux enrichi en l'acide aminé.

Le mélange de départ comprend en outre des sucres et/ou des sels, et dans lequel le flux enrichi en l'acide aminé comporte moins de sucres et/ou de sels que le mélange de départ.

Selon un mode de réalisation, la deuxième étape d'élution est une étape d'élution isocratique.

L'acide aminé présente une constante de basicité Kb et, lors de la première étape, le pH est inférieur ou égal à pKb-6.

Selon un mode de réalisation, la deuxième étape d'élution est effectuée directement à la suite de la première étape de mise en contact, sans rinçage intermédiaire.

La solution d'éluant est une solution d'une base dans l'eau, la base étant de préférence de la soude, de la potasse, de l'ammoniac ou un mélange de ceux-ci, et de manière plus particulièrement préférée de l'ammoniac.

Selon un mode de réalisation, la concentration de la base dans la solution d'éluant est de 0,01 à 50 g/L, de préférence de 0,2 à 20 g/L, plus particulièrement de 0,5 à 10 g/L, et tout particulièrement de 1 à 5 g/L.

Le rapport du nombre de moles de base dans la solution d'éluant utilisée à la deuxième étape sur le nombre de moles d'acide aminé dans le mélange de départ mis en contact à la première étape est inférieur ou égal à 3, de préférence inférieur ou égal à 2, de préférence inférieur ou égal à 1,5, de préférence inférieur ou égal à 1, de préférence inférieur ou égal à 0,9, de préférence inférieur ou égal à 0,8, de préférence inférieur ou égal à 0,7, de préférence inférieur ou égal à 0,6, de préférence inférieur ou égal à 0,5, de préférence inférieur ou égal à 0,4.

Selon un mode de réalisation, l'acide aminé est le tryptophane ou la phénylalanine ou la tyrosine ou l'histidine.

Selon un mode de réalisation, la résine cationique forte est un copolymère polystyrène-divinylbenzène.

Selon un mode de réalisation, la résine cationique forte présente un taux de réticulation de 5 à 12 %, de préférence de 6 à 11 %, plus particulièrement de 7 à 10 %, de préférence de 8 à 9 %.

Selon un mode de réalisation, la résine cationique forte est sous forme cationique monovalente, de préférence sous forme Na⁺ ou K⁺ ou NH₄⁺, et de manière plus particulièrement préférée sous forme NH₄⁺.

Selon un mode de réalisation, le procédé est mis en œuvre dans une installation chromatographique multi-colonnes, l'installation comportant de préférence 30 colonnes au plus, de manière plus particulièrement préférée 20 colonnes au plus, ou 12 colonnes au plus, ou 8 colonnes au plus, ou 6 colonnes au plus.

Selon un mode de réalisation, le procédé est mis en œuvre dans une installation chromatographique à lit non-statique, et de préférence dans une installation chromatographique à lit mobile simulé, ou à lit mobile réel, ou à lit mobile simulé amélioré, ou à lit mobile simulé séquentiel, et de préférence à lit mobile simulé séquentiel.

Selon un mode de réalisation, le mélange de départ est obtenu à l'issu du traitement d'un mélange brut, le mélange brut étant de préférence un moût fermentaire, synthétique ou enzymatique, et ledit traitement comportant de préférence une ou plusieurs étapes choisies parmi une centrifugation, une filtration frontale, une cristallisation, une filtration tangentielle, une microfiltration et une ultrafiltration.

Selon un mode de réalisation, le procédé comprend une ou plusieurs étapes de concentration du flux enrichi en l'acide aminé, de préférence choisies parmi une concentration par évaporation et notamment par évaporation à film tombant, une concentration par évaporation multi-effets, une concentration membranaire et notamment une nanofiltration.

Selon un mode de réalisation, à l'issue de la ou des étapes de concentration, un flux d'acide aminé concentré d'une part et un flux de solution d'éluant d'autre part sont collectés, le procédé comprenant le recyclage de ce flux de solution d'éluant vers la deuxième étape d'élution.

Selon un mode de réalisation, la différence entre le pH de la solution d'éluant utilisée à la deuxième étape et le pH de la première étape est supérieure ou égale à 2, de préférence supérieure ou égale à 4, plus particulièrement supérieure ou égale à 6, et notamment supérieure ou égale à 8.

La présente invention permet de surmonter les inconvénients de l'état de la technique. Elle fournit plus particulièrement un procédé de purification d'acide aminé aromatique amélioré, permettant notamment une séparation entre l'acide aminé cible et des impuretés telles que les sels, les sucres, les couleurs, avec une consommation minime de produits chimiques.

Cela est accompli grâce à l'utilisation d'une résine cationique forte (résine échangeuse d'ions), qui n'est pas utilisée en mode d'échange d'ions. L'invention repose sur un mode d'élution chromatographique où l'acide aminé aromatique est séparé de sa solution initiale par un phénomène de retardation dû à son affinité avec la résine. Ainsi, l'invention prévoit de mettre en contact l'acide aminé sous forme majoritairement zwitterionique avec la résine (pH supérieur ou égal au pKa de l'acide aminé), puis de l'éluer avec une solution de pH plus élevé.

Le mode opératoire diffère des procédés existants qui mettent en œuvre un phénomène de capture par immobilisation de l'acide aminé, puis de désorption par un éluant de régénération concentré.

Sans vouloir être liés par une théorie, les inventeurs estiment que le mécanisme de séparation mis en œuvre est probablement un phénomène d'adsorption par interaction hydrophobe entre les liaisons Pi du groupement aromatique et/ou du doublet du ou des atomes d'azote présents dans le cycle de l'acide aminé, et les fonctions hydrophobes de la matrice de la résine : la séparation ne consiste donc pas en une séparation par échange d'ions.

La présente invention peut notamment être mise en œuvre avec une solution d'élution diluée et avec un ratio éluant / acide aminé plus faible, ce qui permet de minimiser les consommations chimiques et les quantités d'effluents.

L'invention permet également de mettre en œuvre un mécanisme de purification de façon contrôlée, évitant les variations importantes de rendement et de pureté en acides aminés produits.

### BREVE DESCRIPTION DES FIGURES

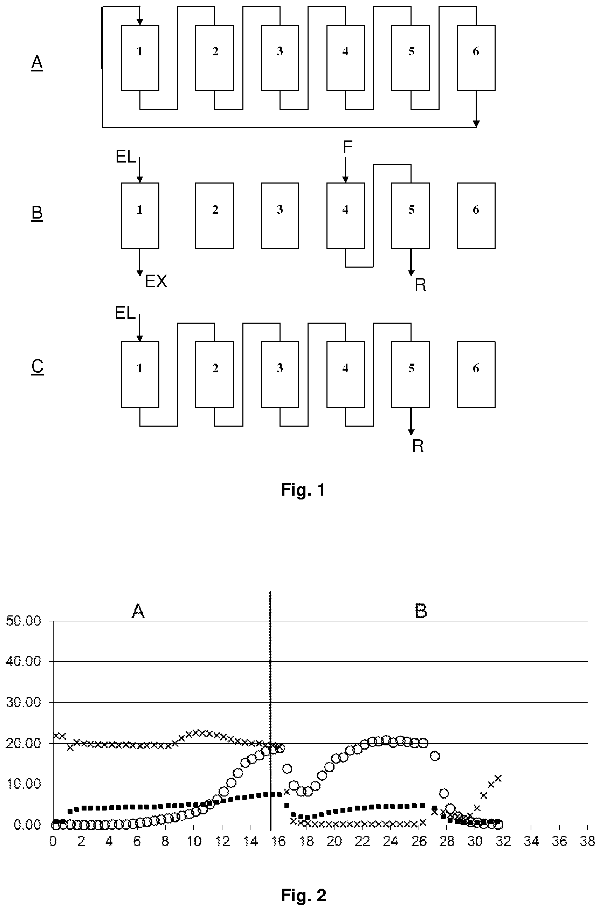

La **figure 1** représente de manière schématique une installation permettant de mettre en œuvre le procédé selon un mode de réalisation de l'invention.
La **figure 2** illustre l'évolution de la concentration de tryptophane (en g/L, symbole O), de la conductivité (en mS/cm, symbole X) et du taux de matière sèche (en degrés Brix, symbole ■) en fonction du volume injecté (en abscisse, en BV ou « Bed Volumes », c'est-à-dire en multiples du volume de lit) dans une colonne de séparation chromatographique. Les conditions opératoires sont précisées dans l'exemple 1.

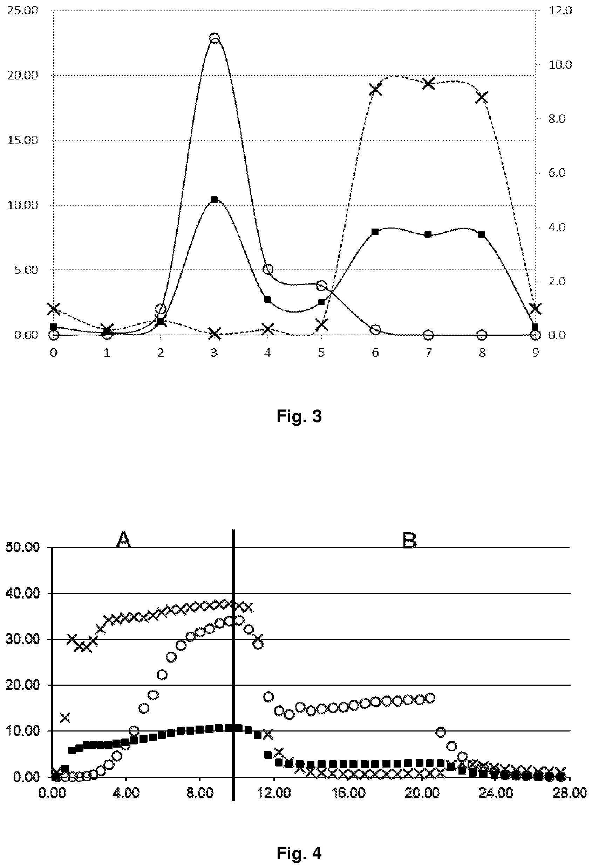

La **figure 3** illustre l'évolution de la concentration de tryptophane (en g/L, échelle de gauche, symbole O), de la conductivité (en mS/cm, échelle de gauche, symbole X) et du taux de matière sèche (en degrés Brix, échelle de droite, symbole ■) sur plusieurs colonnes chromatographiques (en abscisse, le numéro de colonne) dans un système de purification de type SSMB. Les conditions opératoires sont précisées dans l'exemple 2.
La **figure 4** illustre l'évolution de la concentration de phénylalanine (en g/L, symbole O), de la conductivité (en mS/cm, symbole X) et du taux de matière sèche (en degrés Brix, symbole ■) en fonction du volume injecté (en abscisse, en BV) dans une colonne de séparation chromatographique. Les conditions opératoires sont précisées dans l'exemple 3.

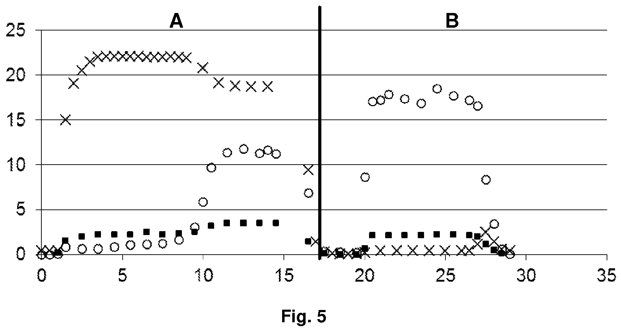

La **figure 5** illustre l'évolution de la concentration d'histidine (en g/L, symbole O), de la conductivité (en mS/cm, symbole X) et du taux de matière sèche (en degrés Brix, symbole ■) en fonction du volume injecté (en abscisse, en BV) dans une colonne de séparation chromatographique. Les conditions opératoires sont précisées dans l'exemple 4**.**

### DESCRIPTION DE MODES DE REALISATION DE L'INVENTION

L'invention est maintenant décrite plus en détail et de façon non limitative dans la description qui suit.

L'invention permet de collecter sous forme purifiée un acide aminé aromatique présent dans un mélange de départ, généralement une solution aqueuse (moût de fermentation, synthétique, ou issu d'enzymation) contenant des impuretés de diverses natures : couleur, sels / minéraux, sucres.

Ainsi, selon un mode de réalisation, le mélange de départ contient des sucres, par exemple des nutriments de fermentation (glucose et/ou saccharose, etc.) et/ou des métabolites. La teneur totale en sucres (et en particulier la teneur en monosaccharides et disaccharides) dans le mélange de départ peut ainsi être par exemple de 0,01 à 100 g/L, et de préférence de 0,1 à 15 g/L.

Selon un mode de réalisation, le mélange de départ contient des sels, qui peuvent être des sels minéraux et/ou des sels organiques. La teneur totale en sels dans le mélange de départ peut ainsi être par exemple de 0,01 à 80 g/L, et de préférence de 0,5 à 20 g/L.

La conductivité du mélange de départ peut être par exemple de 1 à 200 mS/cm, de préférence de 2 à 100 mS/cm, et notamment de 5 à 50 mS/cm.

Selon un mode de réalisation, le mélange de départ est obtenu à l'issue d'un prétraitement par clarification du bouillon de culture (ou d'enzymation). Cette opération de clarification peut être menée par diverses technologies :
- Décantation / centrifugation.
- Filtration tangentielle sur membrane organique, plane ou spiralée, avec un seuil de coupure typiquement compris entre 2000 et 300.000 Da.
- Filtration tangentielle sur membrane céramique, avec un seuil de coupure typiquement supérieur à 10.000 Da.
- Filtration sur filtre inox.
- Combinaison des opérations précédentes, l'ordre pouvant être ajusté selon les cas en fonction de la nature du mélange de départ et des objectifs du procédé.

L'acide aminé aromatique est de préférence la phénylalanine, le tryptophane, la tyrosine, l'histidine, ou un dérivé de ceux-ci. On peut également chercher à récupérer un mélange d'acides aminés aromatiques.

L'invention prévoit une séparation chromatographique sur une résine cationique forte. Par « *résine cationique forte* », on entend une résine ayant des fonctions acides greffées qui sont des acides forts.

Selon un mode de réalisation préféré, la « *résine cationique forte »* signifie une résine comportant des fonctions acide sulfonique.

Selon un mode de réalisation, la résine cationique est une résine polystyrène-divinylbenzène (DVB) forte.

Selon un mode de réalisation, la résine est sous forme de particules, dont le diamètre volumique moyen (Dv50) est de préférence de 20 à 600 µm, notamment de 200 à 350 µm. De préférence, la distribution du diamètre volumique des particules de résine présente un seul maximum (distribution monodisperse). Le coefficient d'uniformité de cette distribution peut être par exemple inférieur ou égal à 1,5, ou inférieur ou égal à 1,3, ou inférieur ou égal à 1,15. La distribution de taille des particules de résine peut être déterminée par granulométrie laser (norme NF 13320).

La résine est de préférence une résine macro-réticulée (et donc n'est pas une résine de type gel). Ainsi, selon des modes de réalisation, le taux de réticulation de la résine (taux de DVB lorsque la résine est le copolymère susmentionné) vaut de 5 à 6 % ; ou de 6 à 7 % ; ou de 7 à 8 % ; ou de 8 à 9 % ; ou de 9 à 10 % ; ou de 10 à 11 % ; ou de 11 à 12 %. On définit le taux de réticulation comme le rapport de la quantité de monomères agents de réticulation (par exemple monomères DVB) sur la quantité totale de monomères (par exemple monomères DVB et autres monomères de la résine) qui sont mis en œuvre dans la fabrication de la résine (les quantités étant exprimées en moles).

Selon un mode de réalisation, la résine est sous forme monovalente. Par exemple, elle peut être sous forme Na⁺, K⁺ ou de préférence NH₄⁺.

Optionnellement, le procédé de l'invention peut comprendre une étape préliminaire de conditionnement de la résine sous une forme ionique souhaitée, notamment sous forme Na⁺, K⁺ ou de préférence NH₄⁺. Cette étape préliminaire peut être effectuée notamment en faisant passer une solution saline ou basique appropriée sur la résine.

Les acides aminés se comportent comme des acides et bases faibles. Ils présentent une constante d'acidité Ka et une constante de basicité Kb. On définit pKa = - log₁₀(Ka) et pKb = - log₁₀(Kb). Le pKa et le pKb représentent les pH de demi-dissociation, c'est-à-dire les pH auxquels la fonction acide, respectivement la fonction basique, des acides aminés est à moitié ionisée.

On appelle zwitterion la forme de l'acide aminé dans laquelle à la fois fonction acide et la fonction amine (basique) sont ionisées.

Les valeurs de pKa et de pKb pour les quatre acides aminés aromatiques susmentionnés sont les suivantes :
- Phénylalanine : pKa = 1,8 et pKb = 9,1.
- Tryptophane : pKa = 2,4 et pKb = 9,4.
- Tyrosine : pKa = 2,2 et pKb = 9,1.
- Histidine : pKa = 1,8 et pKb = 9,2.

L'invention prévoit une étape de mise en contact d'un mélange de départ (ou charge, ou produit d'alimentation) avec une résine chromatographique, puis une étape d'élution.

Dans la première étape, le pH est supérieur ou égal au pKa de l'acide aminé aromatique recherché (ou aux pKa des acides aminés aromatiques recherchés, s'il y en a plusieurs), afin d'assurer que l'acide aminé soit d'une façon significative sous forme de zwitterion.

Selon certains modes de réalisation, le pH lors de la première étape est supérieur ou égal à pKa+0,5 ; ou supérieur ou égal à pKa+1 ; ou supérieur ou égal à pKa+1,5 ; ou supérieur ou égal à pKa+2 ; ou supérieur ou égal à pKa+2,5 ; ou supérieur ou égal à pKa+3.

Il faut noter que le pH lors de la première étape peut également être inférieur ou égal à pKa + 3 ; ou inférieur ou égal à pKa + 2,5 ; ou inférieur ou égal à pKa + 2 ; ou inférieur ou égal à pKa + 1,5 ; ou inférieur ou égal à pKa + 1 ; ou inférieur ou égal à pKa + 0,5.

Le pH lors de la première étape est inférieur ou égal à pKb-6.

Le pH lors de la première étape est compris entre pKa et pKb-6.

Selon certains modes de réalisation, le pH lors de la première étape vaut de 1,8 à 1,9 ; ou de 1,9 à 2,0 ; ou de 2,0 à 2,1 ; ou de 2,1 à 2,2 ; ou de 2,2 à 2,3 ; ou de 2,3 à 2,4 ; ou de 2,4 à 2,5 ; ou de 2,5 à 2,6 ; ou de 2,6 à 2,7 ; ou de 2,7 à 2,8 ; ou de 2,8 à 2,9 ; ou de 2,9 à 3,0 ; ou de 3,0 à 3,2 ; ou de 3,2 à 3,4 ; ou de 3,4 à 3,6 ; ou de 3,6 à 3,8 ; ou de 3,8 à 4,0 ; ou de 4,0 à 4,5 ; ou de 4,5 à 5,0 ; ou de 5,0 à 5,5 ; ou de 5,5 à 6,0 ; ou de 6,0 à 6,5 ; ou de 6,5 à 7,0.

Le pH de la charge (mélange de départ) peut être ajusté avant la mise en contact avec la résine par ajout d'acide ou de base.

Dans la deuxième étape, l'éluant est une solution aqueuse présentant un pH supérieur à celui de la première étape. L'éluant est une solution aqueuse basique, telle qu'une solution de NH₃ ou NaOH notamment.

La différence de pH entre la deuxième étape et la première étape (ΔpH) peut valoir de 0,5 à 1,0 ; ou de 1,0 à 1,5 ; ou de 1,5 à 2,0 ; ou de 2,0 à 2,5 ; ou de 2,5 à 3,0 ; ou de 3,0 à 3,5 ; ou de 3,5 à 4,0 ; ou de 4,0 à 4,5 ; ou de 4,5 à 5,0 ; ou de 5,0 à 5,5 ; ou de 5,5 à 6,0 ; ou de 6,0 à 6,5 ; ou de 6,5 à 7,0 ; ou de 7,0 à 7,5 ; ou de 7,5 à 8,0 ; ou de 8,0 à 8,5 ; ou de 8,5 à 9,0 ; ou de 9,0 à 9,5 ; ou de 9,5 à 10,0 ; ou de 10,0 à 10,5 ; ou de 10,5 à 11,0 ; ou de 11,0 à 11,5 ; ou de 11,5 à 12,0.

Le pH de l'éluant (deuxième étape) peut valoir notamment de 7,0 à 7,5 ; ou de 7,5 à 8,0 ; ou de 8,0 à 8,5 ; ou de 8,5 à 9,0 ; ou de 9,0 à 9,5 ; ou de 9,5 à 10,0 ; ou de 10,0 à 10,5 ; ou de 10,5 à 11,0 ; ou de 11,0 à 11,5 ; ou de 11,5 à 12,0 ; ou de 12,0 à 12,5 ; ou de 12,5 à 13,0 ; ou de 13,0 à 13,5 ; ou de 13,5 à 14,0.

L'éluant peut être une solution d'une base dans l'eau, par exemple NaOH ou NH₃, à une concentration de 0,001 à 0,01 g/L ; ou de 0,01 à 0,1 g/L ; ou de 0,1 à 0,5 g/L ; ou de 0,5 à 1 g/L ; ou de 1 à 2 g/L ; ou de 2 à 3 g/L ; ou de 3 à 4 g/L ; ou de 4 à 5 g/L ; ou de 5 à 6 g/L ; ou de 6 à 7 g/L ; ou de 7 à 8 g/L ; ou de 8 à 9 g/L ; ou de 9 à 10 g/L ; ou de 10 à 15 g/L ; ou de 15 à 20 g/L ; ou de 20 à 25 g/L ; ou de 25 à 50 g/L ; ou de 50 à 100 g/L.

Le nombre de moles de base utilisée pour l'élution, rapporté au nombre de moles d'acide aminé injecté, est inférieur ou égal à 3 ; ou inférieur ou égal à 2 ; ou inférieur ou égal à 1,5 ; ou inférieur ou égal à 1 ; ou inférieur ou égal à 0,9 ; ou inférieur ou égal à 0,8 ; ou inférieur ou égal à 0,7 ; ou inférieur ou égal à 0,6 ; ou inférieur ou égal à 0,5 ; ou inférieur ou égal à 0,4.

L'élution est de préférence isocratique, c'est-à-dire que la composition de l'éluant ne varie pas dans le temps.

Dans un mode de réalisation préféré, le cation de la base utilisée pour l'élution est le cation de la résine. Ainsi, par exemple, si la résine est sous forme Na⁺, l'éluant peut être une solution de NaOH ; si la résine est sous forme K⁺, l'éluant peut être une solution de KOH ; et si la résine est sous forme NH₄⁺, l'éluant peut être une solution d'ammoniac.

L'élution par la base permet de maintenir la forme ionique de la résine, qui pourrait sinon être modifiée par les sels minéraux présents dans solution de charge. En outre, sans vouloir être tenu par une théorie, les inventeurs font l'hypothèse d'un effet de charge osmotique sur la résine, qui tend également à libérer les espèces adsorbées.

L'acide aminé aromatique recherché présente une affinité plus forte pour la résine que la plupart des autres impuretés. On récupère donc les impuretés (par exemple sucres et sels) dans la fraction raffinat, tandis que l'acide aminé est récupéré dans l'extrait.

Selon un mode de réalisation, la teneur massique totale en sucres (en particulier en monosaccharides et disaccharides) dans l'extrait est réduite d'au moins 80 %, de préférence d'au moins 90 %, ou d'au moins 95 %, ou d'au moins 98 %, ou d'au moins 99 %, ou d'au moins 99,5 %, ou d'au moins 99,9 %, par rapport à la teneur massique totale en sucres (en particulier e, monosaccharides et disaccharides) dans le mélange de départ. De préférence, l'extrait (flux enrichi en l'acide aminé) est dépourvu ou essentiellement dépourvu de sucres (en particulier de monosaccharides et disaccharides).

Selon un mode de réalisation, la teneur massique totale en sels dans l'extrait est réduite d'au moins 80 %, de préférence d'au moins 90 %, ou d'au moins 95 %, ou d'au moins 98 %, ou d'au moins 99 %, ou d'au moins 99,5 %, ou d'au moins 99,9 %, par rapport à la teneur massique totale en sels dans le mélange de départ. De préférence, l'extrait (flux enrichi en l'acide aminé) est dépourvu ou essentiellement dépourvu de sels.

Selon un mode de réalisation, certaines impuretés très retenues peuvent être désorbées par une élution complémentaire par un éluant aqueux, salin ou polaire.

Selon un mode de réalisation, aucun rinçage intermédiaire n'est prévu entre l'étape de mise en contact et l'étape d'élution. L'injection de l'éluant suit ainsi directement l'injection du mélange de départ.

Selon un mode de réalisation, l'acide aminé récupéré dans l'extrait issu de la séparation chromatographique subit des traitements complémentaires, tels que :
- Décoloration sur charbon.
- Déminéralisation.
- Concentration sur membrane de type osmose inverse ou nanofiltration.
- Evaporation ou évaporation multi-effets, ce qui permet d'optimiser les consommations énergétiques.
- Cristallisation.
- Combinaison des opérations précédentes, l'ordre pouvant être ajusté selon les cas en fonction de la nature du mélange de départ et les objectifs du procédé.

Selon un mode de réalisation préféré, l'éluant (de préférence une solution de NH₃) est séparé de l'acide aminé et récupéré lors des étapes de post-traitement susmentionnées, afin d'être recyclé en tête du procédé de séparation chromatographique.

Le procédé selon l'invention peut être discontinu (« batch »), semi-continu ou continu. De préférence, il est semi-continu ou continu.

La séparation chromatographique selon l'invention peut être mise en œuvre dans une unité chromatographique à lit statique ou, de préférence, dans une unité chromatographique à lit non-statique.

Dans un système chromatographique à lit statique, le mélange de composés à séparer percole dans une enceinte (ou colonne), généralement cylindrique. La colonne contient un lit de matériau poreux (phase stationnaire) perméable aux fluides. La vitesse de percolation de chaque composé dans le mélange dépend des propriétés physiques du composé. Les composés les plus retenus sur la phase stationnaire percolent plus lentement que les composés les moins retenus sur la phase stationnaire. Ce principe permet d'effectuer la séparation souhaitée.

Il est possible d'effectuer un tel traitement dans plusieurs colonnes en série ou en parallèle, mais généralement une séparation chromatographique dans un système à lit statique est mise en œuvre avec une colonne unique.

Des exemples de tels systèmes chromatographiques à lit statique sont les systèmes HPLC (chromatographie en phase liquide à haute performance) ou CYCLOJET^{™} (système avec recyclage à l'état stationnaire).

Le système CYCLOJET^{™} est tel que décrit dans le document US 6,063,284, auquel il est fait expressément référence. Il s'agit d'un système de séparation chromatographique discontinue à colonne unique, dans lequel les espèces (i) les plus retenues puis (ii) les moins retenues sont collectées séparément à la sortie de la colonne, la portion non-séparée du chromatogramme étant recyclée par la pompe principale, et le mélange à séparer étant périodiquement injecté au moyen d'une boucle d'injection située essentiellement au milieu de la portion recyclée du chromatogramme. Après plusieurs cycles chromatographiques, le procédé atteint un état stationnaire périodique dans lequel la quantité de produits injectés est égale à la quantité de produits séparés collectés séparément à la sortie de la colonne.

Une variante du système CYCLOJET^{™} utilisant deux colonnes est décrite dans le document US 5,630,943, auquel il est fait expressément référence.

Un système à lit non-statique est un système multi-colonnes, dans lequel les positions relatives du lit de phase stationnaire et des points d'injection ou de collecte des flux se déplacent au cours du temps.

Des exemples de tels systèmes chromatographiques à lit non-statique sont les systèmes SMB (lit mobile simulé), iSMB (lit mobile simulé amélioré), SSMB (lit mobile simulé séquentiel), AMB (lit mobile réel), VARICOL^{™}, MODICON^{™}, POWERFEED^{™}, MCSGP ou GSSR (chromatographie de gradient multi-colonnes).

Un système SMB comprend une pluralité de colonnes individuelles contenant un adsorbant, qui sont connectées en série. Un flux d'éluant traverse les colonnes selon une première direction. Les points d'injection du flux d'alimentation et de l'éluant, ainsi que les points de collecte des composés séparés, sont décalés périodiquement et simultanément au moyen d'un ensemble de vannes. L'effet global est de simuler le fonctionnement d'une colonne unique contenant un lit mobile d'adsorbant solide, l'adsorbant solide se déplaçant dans une direction à contre-courant du flux d'éluant. Ainsi, un système SMB est composé de colonnes qui contiennent des lits stationnaires d'adsorbant solide à travers lesquels passe l'éluant, mais le fonctionnement est tel qu'un lit mobile continu à contre-courant est simulé.

La forme la plus conventionnelle d'un système SMB est le système SMB à quatre zones. D'autres formes possibles sont les systèmes SMB à trois zones et les systèmes SMB à deux zones (tels que décrits dans l'article « Two Section Simulated Moving Bed Process » de Kwangnam Lee, dans Séparation Science and Technology 35(4):519-534, 2000, auquel il est fait expressément référence).

Dans les systèmes iSMB et SSMB, il y a au moins une étape dans laquelle le système fonctionne en boucle fermée, sans entrée ou sortie de produit.

Un système iSMB est tel que décrit dans les documents EP 0342629 et US 5,064,539, auxquels il est fait expressément référence.

Un système SSMB découpe les introductions et collectes des flux en sous séquences appliquées de façons périodiques.

D'autres variantes des systèmes SMB sont : le système SMB variant dans le temps et le système POWERFEED^{™}, tels que décrits dans le document US 5,102,553 et dans l'article « PowerFeed operation of simulated moving bed units: changing flow-rates during the switching interval », de Zhang et al. dans Journal of Chromatography A, 1006:87-99, 2003, auxquels il est fait expressément référence; le système MODICON^{™}, tel que décrit dans le document US 7,479,228, auquel il est fait expressément référence ; et le système SMB avec recirculation interne, tel que décrit dans le document US 8,282,831, auquel il est fait expressément référence.

Un système AMB présente un fonctionnement similaire à un système SMB. Toutefois, au lieu de déplacer les points d'injection du flux d'alimentation et de l'éluant, ainsi que des points de collecte, au moyen d'un système de vannes, un ensemble d'unités d'adsorption (colonnes) sont déplacées physiquement par rapport aux points d'alimentation et de collecte. A nouveau, le fonctionnement permet de simuler un lit mobile continu à contre-courant.

Un système de chromatographie VARICOL^{™} est tel que décrit dans les documents US 6,136,198, US 6,375,839 US 6,413,419 et US 6,712,973, auxquels il est fait expressément référence. Un système VARICOL^{™} comprend une pluralité de colonnes individuelles contenant un adsorbant qui sont reliées en série. On fait passer un éluant dans les colonnes selon une première direction. Contrairement au système SMB, les points d'injection pour le mélange à séparer et pour l'éluant et les points de collecte des composés séparés dans le système sont déplacés périodiquement mais de manière asynchrone, au moyen d'un ensemble de vannes. L'effet global est de créer des zones de séparation de longueur variable dans le temps, allouant ainsi la phase stationnaire de manière dynamique dans les zones où elle est la plus utile, et permettant une puissance de séparation similaire avec moins d'unités de séparation chromatographique et une productivité accrue. Contrairement à un système SMB, un système VARICOL^{™} ne simule pas le fonctionnement d'une colonne unique contenant un lit mobile d'adsorbant solide, l'adsorbant solide se déplaçant dans une direction à contre-courant du flux d'éluant, et ainsi le principe de fonctionnement du VARICOL^{™} ne peut pas être mis en œuvre dans un système AMB équivalent.

Un exemple préféré de lit mobile simulé est représenté en référence à la **figure 1**, sous la forme d'un système de chromatographie de type lit mobile simulé séquentiel (SSMB) sur six cellules ou colonnes. Ce système peut être opéré selon un fonctionnement cyclique en quatre phases.
- Phase n°1 (partie A de la figure) : phase de boucle, durant laquelle on maintient une circulation continue en boucle fermée sur toutes les cellules placées en série, pour déplacer le volume interstitiel d'une cellule vers la suivante, sans injection d'éluant.
- Phase n°2 (partie B de la figure) : charge / injection de charge. La charge (F) est injectée en tête de la quatrième cellule. Simultanément, on collecte un volume sensiblement identique de raffinat (R) en sortie de la cinquième cellule. Les cellules 4 et 5 constituent ici la zone 3. Les cellules 2 et 3 constituent la zone de séparation entre extrait et injection de charge. Elles constituent ici la zone 2.
- Phase n°3 (partie B de la figure) : élution de l'extrait. L'éluant (EL) et injecté sur la première cellule pour éluer l'extrait (EX), qui est collecté en volume sensiblement identique au bas de la première cellule. La cellule n°1 constitue ici la zone 1.
- Les phases n°2 et 3 sont de préférence opérées simultanément pour accroître la productivité du système.
- Phase n°4 : élution du raffinat. L'éluant (EL) est injecté en tête de la première cellule, et le raffinat (R) est collecté en volume sensiblement identique sortie de la cinquième. La cellule n°6 est ici une cellule tampon permettant d'assurer la séparation entre la queue de l'extrait et la tête du raffinat. Elle constitue la zone 4. Cette zone peut être omise dans le cas où le degré de pureté et/ou le rendement recherché est relativement limité.

Ces phases sont opérées dans l'ordre dans un mode de réalisation préféré, de 1 à 4. Leur enchaînement constitue une séquence complète.

Chaque séquence (phases n°1 à 4) est répétée six fois en décalant les entrées et sorties de cellules par incrémentation du numéro de cellule, de la gauche vers la droite du système : la charge est ainsi injectée en haut de cellule n°1 en séquence n°1, puis en haut de cellule n°2 en séquence n°2, etc.

Un cycle de production complet est réalisé après achèvement des six séquences successives, quand le point d'injection de la charge, initialement en entrée de cellule n°1, revient de nouveau en entrée de cellule n°1.

Dans ce qui précède, on a donné une description du système SSMB en référence au cas où les cellules correspondent à des colonnes. Ceci n'est pas limitatif, et l'invention s'applique aussi aux systèmes dans lesquels les cellules, ou encore compartiments, sont des parties de colonne.

Par ailleurs, le nombre de colonnes présentes en zones 1, 2, 3 et 4 peut varier en fonction de la qualité de séparation désirée. On peut donc concevoir des systèmes du même type avec une cellule, deux cellules, trois cellules, quatre cellules et jusqu'à douze cellules ou plus.

Le procédé peut encore être mis en œuvre dans une installation multi-colonnes non continue à boucle fermée ou à boucle ouverte, telle que le système DCC décrit dans le brevet WO 2007/012750.

Selon un mode de réalisation, une installation multi-colonnes (notamment du type SMB ou analogue) contient une ou plusieurs zones de nettoyage, permettant de déconnecter une colonne de la boucle de séparation, afin de désorber périodiquement des composés (impuretés) fortement adsorbés, puis de régénérer et rééquilibrer l'adsorbant avec l'éluant de la séparation.

### EXEMPLES

Les exemples suivants illustrent l'invention sans la limiter.

### Exemple 1 - étude de la rétention du tryptophane

Dans cet exemple, on traite un moût de fermentation de tryptophane, prétraité par ajustement à pH = 2,6, puis microfiltration et diafiltration sur membranes céramiques.

Le produit est traité sur une résine Applexion^{®} XA2014-22 sur un volume de 30 mL par colonne (dans cet exemple, 30 mL représente 1BV où BV signifie le volume de lit, ou Bed Volume, de résine). La résine est une résine cationique forte, de type polymérique polystyrène-DVB avec 8 % de DVB, de granulométrie monodisperse 220 µm, et de capacité d'échange ionique 1,8 eq/Lᵣₑₛᵢₙₑ. La résine est préalablement convertie sous la forme Na à l'aide d'une solution de soude à 4 %.

Le mélange de départ pour l'étape de chromatographie contient 17,5 g/L de tryptophane, 3,7 g/L de sucres résiduels, et présente une conductivité de 18,5 mS/cm induite par les sels en solution.

Ce mélange est injecté en excès sur la résine. Lors de la phase d'injection, on observe une différence de comportement des différentes espèces sur la résine :
- Les impuretés salines ne sont pas retenues sur la résine: on estime qu'un phénomène d'exclusion d'ions entre leur charge ionique et les charges de la résine empêche leur pénétration au sein de la matrice. Une conductivité identique à celle du mélange de départ est mesurée en sortie de colonne pendant l'étape d'élution.
- Les sucres, espèces neutres, peuvent pénétrer dans la matrice de la résine, et sont légèrement ralentis, mais non retenus : ils sont co-élués avec les sels pendant la phase d'injection.
- Le tryptophane, injecté majoritairement sous forme zwitterionique à pH = 2,6 subit quant à lui un phénomène de retardation par interaction entre son groupe aromatique et ceux de la résine.

L'élution du tryptophane ainsi retenu est obtenue avec une concentration de soude de 4 g/L, à 2 BV/h.

La **figure 2** représente l'évolution des concentrations en un point du système qui voit se dérouler l'ensemble des étapes de séparation, du début de l'élution du raffinat à la fin de rinçage de l'extrait. La phase A est la phase d'injection du mélange de départ, la phase B est la phase d'élution avec la solution de soude.

Ce système permet d'opérer avec les performances suivantes :
- 205,6 g de tryptophane traité par litre de résine sur un cycle de production de 8 heures ;
- concentration < 100 µS / cm pendant la collecte de l'extrait de tryptophane, correspondant à 99,5 % de déminéralisation.

### Exemple 2 - séparation multi-colonnes du tryptophane

Dans cet exemple, l'invention est appliquée en mode chromatographique sur un système continu et multi-colonnes à un moût de fermentation à pH = 4 contenant du tryptophane à 40 g/L. Les impuretés principales sont des résidus de substrats, des minéraux, ainsi que des résidus solides de métabolisme cellulaire.

Le pH du moût est ici ajusté à 2,7 par ajout de H₂SO₄. A ce pH supérieur au pKa, le tryptophane se trouve sous une forme majoritairement zwitterionique.

Il est ensuite clarifié par microfiltration tangentielle sur une membrane céramique. Les matières solides en suspension sont retenues par la membrane et concentrées dans une fraction dénommée rétentat. Le tryptophane, ainsi que la majorité des impuretés solubles, sont transportés, quant à eux, à travers la membrane vers une fraction appelée perméat.

En fin de concentration, un ajout d'eau dans le compartiment rétentat permet de diluer puis de reconcentrer le rétentat : cette opération, dite de diafiltration, permet d'augmenter le rendement global en tryptophane en diminuant sa teneur résiduelle dans le compartiment rétentat.

Le perméat de microfiltration ainsi traité contient 98 % du tryptophane initialement présent dans le moût, à une concentration de 15 g/L. Les impuretés y sont présentes à hauteur de 25 g/L de sels minéraux : sa conductivité vaut 15,9 mS/cm. On mesure également un taux de sucres de 3 g/L.

Ce perméat est utilisé comme produit d'alimentation du système SSMB décrit ci-dessus.

Suivant le principe du SSMB préalablement exposé, le système utilisé dans ce cas précis est constitué de 9 cellules de séparation, mises en œuvre sous forme de colonnes, et réparties comme suit :
- Zone 1 : trois colonnes = zone d'élution du tryptophane ;
- Zone 2 : deux colonnes = zone de séparation ;
- Zone 3 : trois colonnes = zone de ralentissement du tryptophane ;
- Zone 4 : une colonne = zone de boucle.

Chaque colonne contient 475 mL de résine Applexion^{®} XA2014-22. On définit ainsi la taille d'un volume de lit, ou Bed Volume : 1 BV = 475 mL.

La résine est initialement conditionnée sous forme NH₄⁺ par percolation d'ammoniaque NH₃ de concentration 3 N.

On opère le système à 4,5 BV/h.

Le produit d'alimentation est injecté en tête de zone 3. A chaque période de production sont injectés 7,5 BV de produit d'alimentation. Sur trois colonnes, le tryptophane est ralenti par phénomène de retardation sur la résine, et ainsi séparé des impuretés qui sont éluées et collectées, en sortie de zone, dans une fraction dite « raffinat ». La majorité des impuretés (sels et sucres) sont éluées dans ce raffinat : sa conductivité vaut 17,6 mS/cm.

Le système est réglé de telle manière que 0,1 % du tryptophane initial est élué dans la fraction raffinat.

En tête de zone 1 est injecté de l'ammoniaque dilué à 2 g/L (éluant) qui permet de désorber le tryptophane. A chaque période de production sont injectés 5 BV d'éluant. Le tryptophane est récupéré lors de cette élution dans une fraction dite « *extrait* »*.* La conductivité de l'extrait est inférieure à 200 µS/cm : celui-ci est déminéralisé à hauteur de 98,8 % par rapport au produit d'alimentation. On mesure également une réduction de 89 % des sucres résiduels, et de 75 % de la couleur.

Une très faible quantité d'éluant ammoniacal est utilisé : le ratio éluant / produit d'alimentation vaut 0,67 (volume / volume). Aussi, un phénomène de concentration est observé pendant cette opération : le tryptophane est élué à 20 g/L (contre 15 g/L dans le produit d'alimentation). Plus de 99 % du tryptophane initial est récupéré dans l'extrait au cours de cette étape. A chaque séquence du cycle SSMB, la quantité de NH₃ utilisée est de 0,59 mol par L de résine, pour une quantité de tryptophane injectée de 1,47 mol / L de résine (soit 0,4 mole NH₃ par mole de tryptophane) : ceci démontre que le mécanisme de rétention n'est pas l'échange d'ions.

Le profil mesuré dans les neuf colonnes, en régime permanent, et pendant une étape de boucle, est représenté sur la **figure 3****.**

Y sont représentés les profils de concentrations en tryptophane, de Brix (représentatif de la concentration de matière sèche) et de conductivité (représentatif de la concentration en impuretés salines). On observe ainsi la séparation entre la zone d'élution du tryptophane (zone 1, colonnes n°2, 3 et 4) et la zone de collecte du raffinat (zone 3, colonnes n°7, 8 et 9).

### Exemple 3 - étude de la rétention de la phénylalanine

Cet exemple est mis en œuvre à partir d'un moût de fermentation de phénylalanine, prétraité par ajustement à pH=2, puis microfiltration sur membranes céramiques.

Le produit est traité sur une résine Applexion^{®} XA2014-22, sur un volume de 30 mL par colonne = 1 BV. La résine est préalablement convertie sous la forme NH₄⁺ à l'aide de NH₃ à 3%, rincée, puis équilibrée par passage de NH₃ à 2 g/L.

Le produit d'alimentation de chromatographie contient 32,76 g/L de phénylalanine et présente une conductivité de 36,8 mS/cm due à la concentration saline du milieu.

Le produit d'alimentation est injecté en excès sur la résine, à 4 BV/h : on observe un phénomène de rétention de phénylalanine par rapport aux sels et aux sucres, puis une fuite de l'acide aminé. Une fois la concentration en sortie égale à la concentration d'entrée en acide aminé, l'injection de produit d'alimentation est arrêtée.

L'élution de phénylalanine est faite avec une concentration de NH₃ de 2 g/L, à 4 BV/h.

La **figure 4** représente l'évolution des concentrations en un point du système qui voit se dérouler l'ensemble des étapes de séparation, du début de l'élution raffinat à la fin de rinçage de l'extrait. La phase A est la phase d'injection du mélange de départ, la phase B est la phase d'élution avec la solution de NH₃.

Ce système permet d'opérer avec les performances suivantes :
- 169 g de phénylalanine traité par litre de résine sur un cycle de production de 6,75 heures.
- concentration d'environ 600 µS/cm pendant la collecte de l'extrait de phénylalanine, correspondant à 98,5% de déminéralisation.

### Exemple 4 - étude de la rétention de l'histidine

Cet exemple est mis en œuvre à partir d'une solution d'histidine à 12,98 g/L, de conductivité 17,48 mS/cm et contenant 3,9 g/L de sucres. La solution est préalablement ajustée à pH = 2,7 par ajout d'H₂SO₄.

Le produit est traité sur une résine Applexion^{®} XA2014-22, sur un volume de 17 mL par colonne (= 1 BV). La résine est préalablement convertie sous la forme NH₄⁺ à l'aide de NH₃ à 3%, rincée, puis équilibrée par passage de NH₃ à 2 g/L.

Le produit d'alimentation est injecté en excès sur la résine, à 2 BV/h : on observe un phénomène de rétention de l'histidine, puis une fuite de l'acide aminé. Une fois la concentration en sortie égale à la concentration d'entrée en acide aminé, l'injection de produit d'alimentation est arrêtée.

L'élution de l'histidine est faite avec une concentration de NH₃ de 2 g/L, à 2 BV/h.

La **figure 5** représente l'évolution des concentrations en un point du système qui voit se dérouler l'ensemble des étapes de séparation, du début de l'élution raffinat à la fin de rinçage de l'extrait. La phase A est la phase d'injection du mélange de départ, la phase B est la phase d'élution avec la solution de NH3.

Ce système permet d'opérer avec les performances suivantes :
- 120 g d'histidine traité par litre de résine sur un cycle de production de 30 heures.
- Concentration d'environ 220 µS/cm pendant la collecte de l'extrait de phénylalanine, correspondant à 98,8% de déminéralisation.

## Revendications

1. Procédé de purification d'un acide aminé à partir d'un mélange de départ comprenant en outre des sucres et/ou des sels, dans lequel l'acide aminé comporte un cycle aromatique et présente une constante d'acidité Ka et une constante de basicité Kb, le procédé comprenant :
- une première étape de mise en contact du mélange de départ avec une résine cationique ayant des fonctions acides greffées qui sont des acides forts, à un pH supérieur ou égal à pKa et inférieur ou égal à pKb-6 ;
- une deuxième étape d'élution avec une solution aqueuse d'éluant présentant un pH supérieur au pH de la première étape, permettant de collecter un flux enrichi en l'acide aminé comportant moins de sucres et/ou de sels que le mélange de départ, la solution d'éluant étant une solution d'une base dans l'eau ;
le rapport du nombre de moles de base dans la solution d'éluant utilisée à la deuxième étape sur le nombre de moles d'acide aminé dans le mélange de départ mis en contact à la première étape étant inférieur ou égal à 3,
l'acide aminé étant séparé du mélange de départ par un phénomène de retardation dû à son affinité avec la résine.

2. Procédé selon la revendication 1, dans lequel la deuxième étape d'élution est une étape d'élution isocratique.

3. Procédé selon la revendication 1 ou 2, dans lequel l'acide aminé est le tryptophane ou la phénylalanine ou la tyrosine ou l'histidine.

4. Procédé selon l'une des revendications 1 à 3, dans lequel la deuxième étape d'élution est effectuée directement à la suite de la première étape de mise en contact, sans rinçage intermédiaire.

5. Procédé selon l'une des revendications 1 à 4, dans lequel la base est de la soude, de la potasse, de l'ammoniac ou un mélange de ceux-ci, et de manière plus particulièrement préférée de l'ammoniac ; et la concentration de la base dans la solution d'éluant est de préférence de 0,01 à 50 g/L, de préférence encore de 0,2 à 20 g/L, plus particulièrement de 0,5 à 10 g/L, et tout particulièrement de 1 à 5 g/L.

6. Procédé selon l'une des revendications 1 à 5, dans lequel le rapport du nombre de moles de base dans la solution d'éluant utilisée à la deuxième étape sur le nombre de moles d'acide aminé dans le mélange de départ mis en contact à la première étape est inférieur ou égal à 2, plus particulièrement inférieur ou égal à 1, et idéalement inférieur ou égal à 0,5.

7. Procédé selon l'une des revendications 1 à 6, dans lequel la résine cationique est un copolymère polystyrène-divinylbenzène.

8. Procédé selon l'une des revendications 1 à 7, dans lequel la résine cationique présente un taux de réticulation de 5 à 12 %, de préférence de 6 à 11 %, plus particulièrement de 7 à 10 %, de préférence de 8 à 9 %.

9. Procédé selon l'une des revendications 1 à 8, dans lequel la résine cationique est sous forme cationique monovalente, de préférence sous forme Na⁺ ou K⁺ ou NH₄⁺, et de manière plus particulièrement préférée sous forme NH₄⁺.

10. Procédé selon l'une des revendications 1 à 9, qui est mis en œuvre dans une installation chromatographique multi-colonnes, l'installation comportant de préférence 30 colonnes au plus, de manière plus particulièrement préférée 20 colonnes au plus, ou 12 colonnes au plus, ou 8 colonnes au plus, ou 6 colonnes au plus.

11. Procédé selon l'une des revendications 1 à 10, qui est mis en œuvre dans une installation chromatographique à lit non-statique, et de préférence dans une installation chromatographique à lit mobile simulé, ou à lit mobile réel, ou à lit mobile simulé amélioré, ou à lit mobile simulé séquentiel, et de préférence à lit mobile simulé séquentiel.

12. Procédé selon l'une des revendications 1 à 11, dans lequel le mélange de départ est obtenu à l'issu du traitement d'un mélange brut, le mélange brut étant de préférence un moût fermentaire, synthétique ou enzymatique, et ledit traitement comportant de préférence une ou plusieurs étapes choisies parmi une centrifugation, une filtration frontale, une cristallisation, une filtration tangentielle, une microfiltration et une ultrafiltration.

13. Procédé selon l'une des revendications 1 à 12, comprenant une ou plusieurs étapes de concentration du flux enrichi en l'acide aminé, de préférence choisies parmi une concentration par évaporation et notamment par évaporation à film tombant, une concentration par évaporation multi-effets, une concentration membranaire et notamment une nanofiltration ; et de préférence dans lequel, à l'issue de la ou des étapes de concentration, un flux d'acide aminé concentré d'une part et un flux de solution d'éluant d'autre part sont collectés, le procédé comprenant le recyclage de ce flux de solution d'éluant vers la deuxième étape d'élution .

14. Procédé selon l'une des revendications 1 à 13, dans lequel la différence entre le pH de la solution d'éluant utilisée à la deuxième étape et le pH de la première étape est supérieure ou égale à 2, de préférence supérieure ou égale à 4, plus particulièrement supérieure ou égale à 6, et notamment supérieure ou égale à 8.

## Patentansprüche

1. Verfahren zur Reinigung einer Aminosäure, ausgehend von einer ferner Zucker und/oder Salze umfassenden Ausgangsmischung,
wobei die Aminosäure einen aromatischen Zyklus umfasst und eine Säurekonstante Ka und eine Basizitätskonstante Kb aufweist,
wobei das Verfahren umfasst:
- in einem ersten Schritt: Kontaktieren der Ausgangsmischung mit einem kationischen Harz mit aufgepfropften Säurefunktionen, bei denen es sich um starke Säuren handelt, bei einem pH-Wert größer und gleich pKa und kleiner oder gleich pKb-6;
- in einem zweiten Schritt: Eluieren mit einer wässrigen Elutionsmittellösung mit einem den pH-Wert des ersten Schritts übersteigenden pH-Wert, wodurch ein an Aminosäure angereicherter Fluss erfasst werden kann, der weniger Zucker und/oder Salze enthält als die Ausgangsmischung, wobei es sich bei der Elutionsmittellösung um eine Lösung aus einer Base im Wasser handelt;
wobei das Verhältnis der Anzahl der Mole der Base in der im 2. Schritt verwendeten Elutionsmittellösung zur Anzahl der Mole der Aminosäure in der im 1. Schritt kontaktierten Ausgangsmischung kleiner oder gleich 3 ist,
wobei die Aminosäure infolge ihrer Affinität zum Harz durch eine Verzögerungserscheinung von der Ausgangsmischung getrennt wird.

2. Verfahren nach Anspruch 1, wobei der 2. Elutionsschritt ein isokratischer Elutionsschritt ist.

3. Verfahren nach Anspruch 1 oder 2, wobei es sich bei der Aminosäure um Tryptophan, Phenylalanin, Tyrosin oder Histidin handelt.

4. Verfahren nach einem der Ansprüche 1 - 3, wobei der 2. Elutionsschritt unmittelbar im Anschluss an den 1. Kontaktierschritt ohne Zwischenspülung ausgeführt wird.

5. Verfahren nach einem der Ansprüche 1 - 4, wobei es sich bei der Base um Natrium, Kalium, Ammoniak oder eine Mischung davon, vorzugsweise aber um Ammoniak, handelt, und die Konzentration der Base in der Elutionsmittel vorzugsweise 0,01 - 50 g/L, insbesondere 0,2 - 20 g/L, weiter bevorzugt 0,5 - 10 g/L und besonders bevorzugt 1 - 5 g/L beträgt.

6. Verfahren nach einem der Ansprüche 1 - 5, wobei das Verhältnis der Anzahl der Mole der Base Base in der im 2. Schritt verwendeten Elutionsmittellösung zur Anzahl der Mole der Aminosäure in der im 1. Schritt kontaktierten Ausgangsmischung kleiner oder gleich 2, insbesondere kleiner oder gleich 1 und besonders bevorzugt kleiner oder gleich 0,5 ist.

7. Verfahren nach einem der Ansprüche 1 - 6, wobei das kationische Harz ein Polystyrol-Divinylbenzol-Copolymer ist.

8. Verfahren nach einem der Ansprüche 1 - 7, wobei das kationische Harz einen Vernetzungsgrad von 5 - 12 %, insbesondere 6 - 11 %, vorzugsweise 7 - 10 %, besonders bevorzugt 8 - 9 % aufweist.

9. Verfahren nach einem der Ansprüche 1 - 8, wobei das kationische Harz in Form eines einwertigen Kations, vorzugsweise in Form von Na⁺ oder K⁺ oder NH₄⁺, und besonders bevorzugt in Form von NH₄⁺ vorliegt.

10. Verfahren nach einem der Ansprüche 1 - 9, das in einer mehrere Säulen umfassenden Chromatographieeinrichtung ausgeführt wird,
wobei die Einrichtung vorzugsweise höchstens 30, insbesondere höchstens 20, höchstens 12, höchstens 8 oder höchstens 6 Säulen aufweist.

11. Verfahren nach einem der Ansprüche 1 - 10, das in einer Chromatographieeinrichtung mit nicht statischem Fließbett, vorzugsweise in einer Chromatographieeinrichtung mit simuliertem Bewegtbett oder echtem Bewegtbett oder verbessertem Bewegtbett oder sequentiellem simuliertem Bewegtbet, besonders bevorzugtweise mit sequentiellem simuliertem Bewegtbett ausgeführt wird.

12. Verfahren nach einem der Ansprüche 1 - 11, wobei die Ausgangsmischung aus der Bearbeitung einer Rohmasse resultiert, wobei es sich bei der Rohmasse vorzugsweise um eine fermentative, synthetische oder enzymatische Würze handelt und wobei die Bearbeitung vorzugsweise mindestens einen aus folgender Gruppe gewählten Schritt umfasst: Zentrifugation, frontale Filtration, Kristallisation, tangentiale Filtration, Mikrofiltration und Ultrafiltration.

13. Verfahren nach einem der Ansprüche 1 - 12, umfassend mindestens einen Schritt des Konzentrierens des an Aminosäure angereicherten Flusses, der vorzugsweise aus folgender Gruppe gewählt ist: Konzentrieren mittels Verdampfung, inbesondere Fallfilmverdampfung, Konzentrieren mittels Verdampfung mit mehrfacher Wirkung, Membrankonzentration und insbesondere Nanofiltration, vorzugsweise wobei am Ende des mindestens einen Konzentrationsschritts ein konzentrierter Aminosäurefluss einerseits und ein Elutionsmittellösungsfluss andererseits gesammelt werden, wobei das Verfahren Wiederverwenden des Elutionsmittellösungsflusses im zweiten Elutionsschritt umfasst.

14. Verfahren nach einem der Ansprüche 1 - 13, wobei der Unterschied zwischen dem pH-Wert der im 2. Schritt verwendeten Elutionsmittellösung und dem pH-Wert des 1. Schritts größer oder gleich 2, insbesondere größer oder gleich 4, vorzugsweise größer oder gleich 6 und besonders bevorzugt größer oder gleich 8 ist.

## Claims

1. A method for purifying an amino acid from an initial mixture further comprising sugars and/or salts, wherein the amino acid includes an aromatic ring and has an acidity constant Ka and a basicity constant Kb, the method comprising:
- a first step of putting the initial mixture into contact with a cation resin having grafted acid functions which are strong acids, at a pH greater than or equal to pKa and lower than or equal to pKb-6;
- a second elution step with an aqueous solution of eluent having a pH greater than the pH of the first step, making it possible to collect a flow enriched in the amino acid comprising less sugars and/or salts than the initial mixture, the eluent solution being a solution of a base in water;
the ratio of the number of moles of base in the eluent solution used in the second step over the number of moles of amino acid in the initial mixture contacted in the first step being less than or equal to 3,
the aromatic amino acid being separated from the initial mixture by a delay phenomenon due to its affinity with the resin.

2. The method according to claim 1, wherein the second elution step is an isocratic elution step.

3. The method according to claim 1 or 2, wherein the amino acid is tryptophan or phenylalanine or tyrosine or histidine.

4. The method according to one of claims 1 to 3, wherein the second elution step is directly carried out following the first contacting step without any intermediate rinsing.

5. The method according to one of claims 1 to 4, wherein the base is soda, potash, ammonia or a mixture thereof, and more preferably ammonia ; and the concentration of the base in the eluent solution is preferably from 0.01 to 50 g/L, more preferably from 0.2 to 20 g/L, more particularly from 0.5 to 10 g/L, and most particularly from 1 to 5 g/L.

6. The method according to one of claims 1 to 5, wherein the ratio of the number of moles of base in the eluent solution used in the second step over the number of moles of amino acid in the initial mixture contacted in the first step is less than or equal to 2, more particularly less than or equal to 1, and ideally less than or equal to 0.5.

7. The method according to one of claims 1 to 6, wherein the cation resin is a polystyrene-divinylbenzene copolymer.

8. The method according to one of claims 1 to 7, wherein the cation resin has a crosslinking rate from 5 to 12 %, preferably from 6 to 11 %, more particularly from 7 to 10 %, preferably from 8 to 9 %.

9. The method according to one of claims 1 to 8, wherein the cation resin is in a monovalent cationic form, preferably in the form of Na⁺ or K⁺ or NH₄⁺, and more preferably in the form of NH₄⁺.

10. The method according to one of claims 1 to 9, which is carried out in a multi-column chromatographic installation, the installation preferably including at most 30 columns, more preferably at most 20 columns, or at most 12 columns, or at most 8 columns, or at most 6 columns.

11. The method according to one of claims 1 to 10, which is carried out in a chromatographic installation with a non-static bed, and preferably in a chromatographic installation with a simulated moving bed, or with an actual moving bed, or with an improved simulated moving bed, or with a sequential simulated moving bed, and preferably with a sequential simulated moving bed.

12. The method according to one of claims 1 to 11, wherein the initial mixture is obtained at the end of the treatment of a raw mixture, the raw mixture being preferably a fermentative, synthetic or enzymatic must, and said treatment preferably including one or several steps selected from centrifugation, dead-end filtration, crystallization, tangential filtration, microfiltration and ultrafiltration.

13. The method according to one of claims 1 to 12, comprising one or several steps for concentrating the flow enriched in the amino acid, preferably selected from concentration by evaporation and notably by evaporation with a falling film, concentration by multi-effect evaporation, membrane concentration and notably nanofiltration; and wherein preferably, at the end of the concentration step(s), a concentrated flow of amino acid on the one hand and a flow of eluent solution on the other hand are collected, the method comprising the recycling of this flow of eluent solution to the second elution step.

14. The method according to one of claims 1 to 13, wherein the difference between the pH of the eluent solution used in the second step and the pH of the first step is greater than or equal to 2, preferably greater than or equal to 4, more particularly greater than or equal to 6, and notably greater than or equal to 8.
